# EUROPÄISCHE PATENTSCHRIFT

(11) **EP 0 523 671 B1**
(45) Veröffentlichungstag und Bekanntmachung des Hinweises auf die Patenterteilung: **17.01.1996**
(21) Anmeldenummer: 92112099.4
(22) Anmeldetag: 15.07.1992
(51) Int. Cl.: C07C 205/12

(54) **Verfahren zur Herstellung von Chlorfluornitrobenzolen**
Process for the preparation of chlorofluoronitrobenzenes
Procédé de préparation de chlorofluoronitrobenzènes

(30) Priorität: 17.07.1991 DE 4123600
(43) Veröffentlichungstag der Anmeldung: 20.01.1993
(73) Patentinhaber: HOECHST AKTIENGESELLSCHAFT, D-65926 Frankfurt am Main (DE)
(72) Erfinder: Papenfuhs, Theodor, Dr., W-6000 Frankfurt am Main 50 (DE); Kanschik-Conradsen, Andreas, Dr., W-6084 Gernsheim (Rhein) (DE); Pressler, Wilfried, Dr., W-6233 Kelkheim/Taunus (DE)

(56) Entgegenhaltungen:
- GB-A- 2 042 507
- GB-A- 2 058 067
- US-A- 4 069 262
- US-A- 4 140 719
- CHEMICAL ABSTRACTS, vol. 113, no. 17, 1990, Columbus, Ohio, US; abstract no. 152000q, CHEN, WENHUI 'Prepartion of chlorofluoronitrobenzene' Seite 738;

## Beschreibung

Gegenstand der vorliegenden Erfindung ist ein verbessertes Verfahren zur Herstellung von Chlorfluornitrobenzolen in hohen Ausbeuten durch Umsetzung von Dichlornitrobenzolen mit Alkalimetallfluoriden in Gegenwart eines Katalysators und einer geringen Menge eines Lösungsmittels, dessen Siedepunkt bei den gewählten Druckbedingungen unterhalb der Reaktionstemperatur liegt. Chlorfluornitrobenzole sind wichtige Zwischenprodukte zur Herstellung von Pharmazeutika und Pflanzenschutzmitteln.

Die GB-A 2 058 067 betrifft ein Verfahren zur Herstellung fluorierter aromatischer Verbindungen, die ein oder zwei Fluoratome am Ring und wenigstens eine weitere funktionelle Gruppe enthalten, durch Umsetzung einer entsprechend chlorierten oder bromierten Verbindung mit Kaliumfluorid und einem aprotischen Lösungsmittel in Anwesenheit einer quaternären Ammoniumverbindung. Es wird nicht empfohlen, Kaliumfluorid mit einem bestimmten Wassergehalt zu verwenden und bei einer Reaktionstemperatur, die über dem jeweiligen Siedepunkt des verwendeten aprotischen Lösungsmittel liegt, zu arbeiten.

Die US-PS 4 140 719 betrifft die Herstellung von 2,4-Difluoranilin durch Umsetzung von 2,4,5-Trichlornitrobenzol mittels Kaliumfluorids zu 2,4-Difluor-5-chlornitrobenzol und nachfolgende Hydrierung. Es wird ausdrücklich erwähnt, daß die Fluorierung unter wasserfreien Bedingungen durchgeführt werden soll. Die Fluorierung erfolgt, wie die Beispiele zeigen, stets mit sehr großen Mengen Lösungsmittel, wobei die Reaktionstemperatur unter dem jeweiligen Siedepunkt des verwendeten Lösungsmittels liegt.

In der US-PS 4 164 517 wird die Umsetzung von 3,4- und 2,4-Dichlornitrobenzolen mit vorgetrocknetem Kaliumfluorid in dipolar aprotischen Lösungsmitteln bei Temperaturen oberhalb von 200°C beschrieben. Dabei läßt sich die Ausbeute mit zunehmendem Anteil des Lösungsmittels erhöhen. So wird ausgeführt, daß bei der Umsetzung von 2,4-Dichlornitrobenzol ohne Lösungsmittel selbst nach einer Reaktionszeit von 30 Stunden bei 240°C nur 20 % umgesetzt werden.

In der DE-PS 29 38 939 wird ein Verfahren zur Herstellung von Monofluornitrobenzolen aus insbesondere Monochlornitrobenzolen mit fein gepulvertem Kaliumfluorid in der Schmelze bei vorzugsweise 140 bis 150°C unter Zusatz von Tetraalkyl- oder Arylalkylammoniumsalzen als Katalysator beschrieben. Dabei betragen die Reaktionszeiten gemäß den Beispielen 24 bis 28 Stunden. Die Nachteile dieses Verfahrens sind außer in der schlechten Raum-Zeit-Ausbeute darin zu sehen, daß das vorzugsweise verwendete Kaliumfluorid einen Wassergehalt von weniger als 0,2 Gew.-% enthält, was eine Vorbehandlung des Kaliumfluorids erforderlich macht.

Es bestand daher ein erhebliches Interesse für ein technisch günstigeres Verfahren zur Herstellung von Chlorfluorbenzolen.

Es wurde nun überraschenderweise gefunden, daß man Chlorfluornitrobenzole in hohen Ausbeuten vorteilhaft herstellen kann, indem man Dichlornitrobenzole mit Alkalimetallfluoriden eines Wassergehaltes bis zu 2,5 Gew.-% in Gegenwart eines quartären Ammonium- und/oder Phosphoniumsalzes, Kronenethers und/oder Polyethylenglycoldimethylethers als Katalysator in Anwesenheit eines aprotischen Lösungsmittels, dessen Siedepunkt bei den gewählten Druckbedingungen unterhalb der Reaktionstemperatur liegt, bei Temperaturen von 125°C bis 200°C, vorzugsweise von 140 bis 190°C umsetzt.

Als Ausgangsverbindungen werden vorzugsweise 3,4-Dichlornitrobenzol, 2,3-Dichlornitrobenzol oder 2,5-Dichlornitrobenzol eingesetzt.

An Alkalimetallfluoriden werden vorzugsweise Kaliumfluorid, Rubidiumfluorid oder Cäsiumfluorid oder Kombinationen aus diesen verwendet. Vorteilhaft beim erfindungsgemäßen Verfahren ist, daß Alkalimetallfluoride eingesetzt werden können, deren Wassergehalt bis zu 2,5 Gew.-% betragen kann, so daß beispielsweise technisches Kaliumfluorid ohne Vorbehandlung eingesetzt werden kann.

Als Katalysatoren können quartäre Ammoniumverbindungen, insbesondere Tetraalkyl(C₁-C₂₂)-ammoniumhalogenide, wie Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumbromid; Tetraarylammoniumhalogenide, wobei die Arylreste beispielsweise Phenyl- oder Naphthylreste sein können, die durch Halogenatome, verzweigte oder unverzweigte Alkyl-, Nitro-, Cyano-, Amino- und/oder Alkoxygruppen substituiert sein können; oder gemischte Alkylarylammoniumhalogenide; ferner quartäre Phosphoniumverbindungen, insbesondere Tetraalkyl(C₁-C₂₂)-phosphoniumhalogenide, wie Stearyltributylphosphoniumbromid, Hexadecyltriethylphosphoniumbromid, Hexadecyltributylphosphoniumbromid; Tetraarylphosphoniumhalogenide, wobei die Arylreste beispielsweise Phenyl- oder Naphthylreste sein können, die durch Halogenatome, verzweigte oder unverzeigte Alkyl-, Nitro-, Cyano- und/oder Alkoxygruppen substituiert sein können, oder gemischte Alkylarylphosphoniumhalogenide oder Kronenether (siehe Angewandte Chemie 84 (1972), 16-26, und Römpp's Chemielexikon, 8. Auflage (1983)), wie 18-Krone-6, Polyethylenglycoldimethylether und Kombinationen aus diesen in katalytischen Mengen verwendet werden.

Die vorstehend genannten Katalysatoren werden beim erfindungsgemäßen Verfahren in Mengen von 1 bis 10 gew.-%, vorzugsweise von etwa 2 bis etwa 5 Gew.-%, bezogen auf Dichlornitrobenzol, eingesetzt.

Was das Mengenverhältnis Dichlornitrobenzol zu Alkalimetallfluorid anbelangt, so werden zweckmäßigerweise 1,05 bis 1,7 Mol Dichlornitrobenzol mit 1 Mol Alkalimetallfluorid umgesetzt. Das Dichlornitrobenzol kann aber auch in einem molaren Überschuß bis 5:1 zur Anwendung gelangen. Durch Anwendung des Dichlornitrobenzols im Überschuß kann die Ausbeute an Chlorfluornitrobenzol wesentlich gesteigert werden.

Was die aprotischen Lösungsmittel anbelangt, so wird es durch das erfindungsgemäße Verfahren ermöglicht, Lösungsmittel der genannten Art zu verwenden, deren Siedepunkt bei den gewählten Druckbedingungen unterhalb der Reaktionstemperatur liegen, wodurch sie während der Reaktion kontinuierlich abdestilliert werden können. Dies hat den Vorteil, daß sowohl im Reaktionsgemisch vorhandene als auch während der Reaktion entstehende Verbindungen mit Siedepunkten unterhalb der Reaktionstemperatur mit dem Lösungsmittel aus dem Reaktionsgefäß entfernt und außerhalb des Reaktionsgefäßes kondensiert werden können, was zu einer verringerten Belastung der Abluft führt. Geeignete aprotische Lösungsmittel sind beispielsweise Dimethylsulfoxid, Dimethylacetamid, Dimethylformamid, insbesondere aber Xylol, o-Dichlorbenzol oder 2-Chlortoluol.

Das Verfahren kann sowohl bei Atmosphärendruck als auch bei Über- oder Unterdruck durchgeführt werden. Dies hat zur Folge, daß beispielsweise bei Unterdruck vorteilhaft auch solche aprotischen Lösungsmittel eingesetzt werden können, die bei Atmosphärendruck oberhalb der Reaktionstemperatur sieden, und bei Überdruck vorteilhaft auch solche, die bei Atmosphärendruck verhältnismäßig weit unterhalb der Reaktionstemperatur sieden.

Die aprotischen Lösungsmittel werden beim erfindungsgemäßen Verfahren in nur relativ kleinen Mengen eingesetzt. Es ist zweckmäßig, das Lösungsmittel in einer Menge von 2 bis 15 Molprozent, bezogen auf eingesetztes Dichlornitrobenzol, zu verwenden.

Bei dem erfindungsgemäßen Verfahren ist es wichtig, daß während der gesamten Reaktion eine gute Durchmischung der Reaktionssuspension gewährleistet wird.

Die nachstehenden Beispiele dienen zur Erläuterung der Erfindung, ohne sie darauf zu beschränken.

### Beispiel 1

100 g Octadecyltrimethylammoniumchlorid werden bei 60°C in 2900 g (15,1 Mol) 3,4-Dichlornitrobenzol gelöst. Der Lösung werden 100 g 2-Chlortoluol zugesetzt. Nach Zugabe von 825 g (14,2 Mol) Kaliumfluorid wird die erhaltene Suspension 11 Stunden auf 180°C erwärmt. Während dieser Zeit wird ein Teil des 2-Chlortoluols kontinuierlich abdestilliert. Anschließend wird die Reaktionssuspension scharf abgesaugt, der Filterkuchen mit 2-Chlortoluol gewaschen und die vereinigten Filtrate im Vakuum fraktioniert. Man erhält so 1966 g (79 % der Theorie) 3-Chlor-4-fluornitrobenzol und 340 g nicht umgesetztes 3,4-Dichlornitrobenzol.

### Beispiel 2

70 g Octadecyltrimethylammoniumchlorid werden bei 70°C in 2020 g (10,5 Mol) 3,4-Dichlornitrobenzol und 100 g o-Dichlorbenzol gelöst. Nach Zugabe von 580 g (10 Mol) Kaliumfluorid wird die erhaltene Suspension 11 Stunden auf 180°C unter Rühren und Stickstoff erhitzt. Während dieser Zeit wird ein Teil des o-Dichlorbenzols kontinuierlich abdestilliert. Anschließend wird scharf abgesaugt, der Filterkuchen mit o-Dichlorbenzol gewaschen und die vereinigten Filtrate im Vakuum fraktioniert. Man erhält so 1349 g (77 % der Theorie) 3-Chlor-4-fluornitrobenzol und 246 g nicht umgesetztes 3,4-Dichlornitrobenzol.

### Beispiel 3

20 g Tetramethylammoniumchlorid werden bei 70°C in 100 g 2-Chlortoluol gelöst. Der Lösung werden 1230 g (6,5 Mol) 2,3-Dichlornitrobenzol zugesetzt. Nach Zugabe von 290 g (5 Mol) Kaliumfluorid wird die erhaltene Suspension 14 Stunden auf 180°C unter Rühren und Stickstoff erhitzt. Während dieser Zeit wird ein Teil des 2-Chlortoluols kontinuierlich abdestilliert. Anschließend wird scharf abgesaugt, der Filterkuchen mit 2-Chlortoluol gewaschen und die vereinigten Filtrate im Vakuum fraktioniert. Man erhält so 505 g (57 % der Theorie) 2-Fluor-3-chlornitrobenzol und 437 g nicht umgesetztes 2,3-Dichlornitrobenzol.

### Beispiel 4

25 g Hexadecyltributylphosphoniumbromid werden bei 60°C in 1010 g (5,25 Mol) 3,4-Dichlornitrobenzol gelöst. Der Lösung werden 100 g 2-Chlortoluol zugesetzt. Nach Zugabe von 290 g (5,0 Mol) Kaliumfluorid wird die erhaltene Suspension 16 h auf 200°C erwärmt. Während dieser Zeit wird ein Teil des 2-Chlortoluols kontinuierlich abdestilliert. Anschließend wird die Reaktionssuspension scharf abgesaugt, der Filterkuchen mit 2-Chlortoluol gewaschen und die vereinigten Filtrate im Vakuum fraktioniert. Man erhält so 711 g (81 % der Theorie) 3-Chlor-4-fluornitrobenzol und 115 g nicht umgesetztes 3,4-Dichlornitrobenzol.

## Patentansprüche

1. Verfahren zur Herstellung von Chlorfluornitrobenzolen, wobei man Dichlornitrobenzole mit Alkalimetallfluoriden eines Wassergehalts bis zu 2,5 Gew.-% in Gegenwart eines quartären Ammonium- und/oder Phosphoniumsalzes, Kronenethers und/oder Polyethylenglycoldimethylethers als Katalysator in Anwesenheit eines aprotischen Lösungsmittels, dessen Siedepunkt bei den gewählten Druckbedingungen unterhalb der Reaktionstemperatur liegt, bei Temperaturen von 125 bis 200°C umsetzt.

2. Verfahren nach Anspruch 1, wobei man bei Temperaturen von 140 bis 190°C umsetzt.

3. Verfahren nach mindestens einem der Ansprüche 1 und 2, wobei man als quartäre Ammoniumsalze Tetraalkyl(C₁-C₂₂)-ammoniumhalogenide, Tetraarylammoniumhalogenide, wobei die Arylreste substituiert sein können, und/oder gemischte Alkylarylammoniumhalogenide, als quartäre Phosphoniumsalze Tetraalkyl(C₁-C₂₂)-phosphoniumhalogenide, Tetraarylphosphoniumhalogenide, wobei die Arylreste substituiert sein können, und/oder gemischte Alkylarylphosphoniumhalogenide und als Kronenether 18-Krone-6 einsetzt.

4. Verfahren nach mindestens einem der Ansprüche 1 bis 3, wobei man als Katalysator Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumbromid, Stearyltributylphosphoniumbromid, Hexadecyltriethylphosphoniumbromid und/oder Hexadecyltributylphosphoniumbromid einsetzt.

5. Verfahren nach mindestens einem der Ansprüche 1 bis 4, wobei man den Katalysator in Mengen von 2 bis 10 Gew.-%, bezogen auf Dichlornitrobenzol, einsetzt.

6. Verfahren nach mindestens einem der Ansprüche 1 bis 5, wobei man den Katalysator in Mengen von 2 bis 5 Gew.-%, bezogen auf Dichlornitrobenzol, einsetzt.

7. Verfahren nach mindestens einem der Ansprüche 1 bis 6, wobei man als Alkalimetallfluorid Kaliumfluorid, Rubidiumfluorid oder Cäsiumfluorid oder Kombinationen daraus einsetzt.

8. Verfahren nach mindestens einem der Ansprüche 1 bis 7, wobei man 1,05 bis 1,7 Mol Dichlornitrobenzol mit 1 Mol Alkalimetallfluorid umsetzt.

9. Verfahren nach mindestens einem der Ansprüche 1 bis 7, wobei man 1,05 bis 5 Mol Dichlornitrobenzol mit 1 Mol Alkalimetallfluorid umsetzt.

10. Verfahren nach mindestens einem der Ansprüche 1 bis 9, wobei man als aprotisches Lösungsmittel Xylol, o-Dichlorbenzol, 2-Chlortoluol, Dimethylsulfoxid, Dimethylacetamid oder Dimethylformamid verwendet.

11. Verfahren nach mindestens einem der Ansprüche 1 bis 10, wobei man 2 bis 15 Molprozent aprotisches Lösungsmittel, bezogen auf eingesetztes Dichlornitrobenzol, einsetzt.

12. Verfahren nach mindestens einem der Ansprüche 1 bis 11, wobei während der gesamten Reaktion eine gute Durchmischung der Reaktionssuspension erfolgt.

13. Verfahren nach mindestens einem der Ansprüche 1 bis 12, wobei bei Atmosphärendruck, Überdruck oder Unterdruck gearbeitet wird.

## Claims

1. A process for the preparation of chlorofluoronitrobenzenes, which comprises reacting dichloronitrobenzenes with alkali metal fluorides having a water content of up to 2.5 % by weight in the presence of a quaternary ammonium and/or phosphonium salt, a crown ether and/or polyethylene glycol dimethyl ether as catalyst in the presence of an aprotic solvent, the boiling point of which is below the reaction temperature under the pressure conditions chosen, at temperatures of 125 to 200°C.

2. The process as claimed in claim 1, wherein the reaction is carried out at temperatures of 140 to 190°C.

3. The process as claimed in at least one of claims 1 and 2, wherein the quaternary ammonium salts used are tetra-C₁-C₂₂-alkylammonium halides, tetraarylammonium halides, where the aryl radicals can be substituted, and/or mixed alkylarylammonium halides, the quaternary phosphonium salts used are tetra-C₁-C₂₂-alkylphosphonium halides, tetraarylphosphonium halides, where the aryl radicals can be substituted, and/or mixed alkylarylphosphonium halides and the crown ether used is 18-crown-6.

4. The process as claimed in at least one of claims 1 to 3, wherein the catalyst used is octadecyltrimethylammonium chloride, distearyldimethylammonium chloride, tetramethylammonium chloride, tetramethylammonium bromide, hexadecyltrimethylammonium chloride, benzyltrimethylammonium bromide, stearyltributylphosphonium bromide, hexadecyltriethylphosphonium bromide and/or hexadecyltributylphosphonium bromide.

5. The process as claimed in at least one of claims 1 to 4, wherein the catalyst is used in amounts of 2 to 10 % by weight, relative to dichloronitrobenzene.

6. The process as claimed in at least one of claims 1 to 5, wherein the catalyst is used in amounts of 2 to 5 % by weight, relative to dichloronitrobenzene.

7. The process as claimed in at least one of claims 1 to 6, wherein the alkali metal fluoride used is potassium fluoride, rubidium fluoride or cesium fluoride or combinations thereof.

8. The process as claimed in at least one of claims 1 to 7, wherein 1.05 to 1.7 mol of dichloronitrobenzene are reacted with 1 mol of alkali metal fluoride.

9. The process as claimed in at least one of claims 1 to 7, wherein 1.05 to 5 mol of dichloronitrobenzene are reacted with 1 mol of alkali metal fluoride.

10. The process as claimed in at least one of claims 1 to 9, wherein the aprotic solvent used is xylene, o-dichlorobenzene, 2-chlorotoluene, dimethyl sulfoxide, dimethylacetamide or dimethylformamide.

11. The process as claimed in at least one of claims 1 to 10, wherein 2 to 15 mol percent of aprotic solvent are used, relative to the dichloronitrobenzene used.

12. The process as claimed in at least one of claims 1 to 11, wherein good mixing of the reaction suspension is carried out during the entire reaction.

13. The process as claimed in at least one of claims 1 to 12, wherein atmospheric pressure, overpressure or underpressure is employed.

## Revendications

1. Procédé pour la préparation de chlorofluoronitrobenzènes en faisant réagir les dichloronitrobenzènes avec des fluorures de métaux alcalins ayant une teneur en eau allant jusqu'à 2,5 % en poids, en présence d'un sel d'ammonium et/ou de phosphonium quaternaire, d'un éther couronne et/ou d'un éther diméthylique de polyéthyèneglycol comme catalyseur en présence d'un solvant aprotique, dont le point d'ébullition dans les conditions de pressions choisies est inférieur à la température de réaction, à des températures de 125 à 200 °C.

2. Procédé selon la revendication 1, en effectuant la réaction à des températures de 140 à 190 °C.

3. Procédé selon au moins l'une des revendications 1 et 2, utilisant comme sel d'ammonium quaternaire les halogénures de tétra(alkyle en C₁-C₂₂)ammonium,les halogénures de tétraarylammonium, les radicaux aryle pouvant être substitués et/ou des halogénures d'alkylarylammonium mixtes, comme sels de phosphonium quaternaire, les halogénures de tétra(alkyle en C₁-C₂₂)phosphonium, les halogénures de tétraarylphosphonium, les radicaux aryle pouvant être substitués et/ou des halogénures d'alkylarylphosphonium mixtes, et comme éther couronne le 18-couronne-6.

4. Procédé selon au moins l'une des revendications 1 à 3, utilisant comme catalyseur le chlorure d'octadécyltriméthylammonium, le chlorure de distéaryldiméthylammonium, le chlorure de tétraméthylammonium, le bromure de tétraméthylammonium, le chlorure d'hexadécyltriméthylammonium, le bromure de benzyltriméthylammonium, le bromure de stéaryltributylphosphonium, le bromure d'hexadécyltriéthylphosphonium et/ou le bromure d'hexadécyltributylphosphonium.

5. Procédé selon au moins l'une des revendications 1 à 4, utilisant le catalyseur en quantité de 2 à 10 % en poids par rapport au dichloronitrobenzène.

6. Procédé selon au moins l'une des revendications 1 à 5, utilisant le catalyseur en quantité de 2 à 5 % en poids par rapport au dichloronitrobenzène.

7. Procédé selon au moins l'une des revendications 1 à 6, utilisant comme fluorure de métal alcalin le fluorure de potassium, le fluorure de rubidium ou le fluorure de césium ou leurs combinaisons.

8. Procédé selon au moins l'une des revendications 1 à 7, en faisant réagir de 1,05 à 1,7 mole de dichloronitrobenzène avec 1 mole de fluorure de métal alcalin.

9. Procédé selon au moins l'une des revendications 1 à 7, faisant réagir de 1,05 à 5 moles de dichloronitrobenzène avec 1 mole de fluorure de métal alcalin.

10. Procédé selon au moins l'une des revendications 1 à 9, utilisant comme solvant aprotique le xylène, le o-dichlorobenzène, le 2-chlorotoluène, le diméthylsulfoxyde, le diméthylacétamide ou le diméthylformamide.

11. Procédé selon au moins l'une des revendications 1 à 10, utilisant de 2 à 15 % en moles de solvant aprotique par rapport à la quantité de dichlorobenzène utilisée.

12. Procédé selon au moins l'une des revendications 1 à 11, effectuant au cours de la totalité de la réaction un bon brassage de la suspension réactionnelle.

13. Procédé selon au moins l'une des revendications 1 à 12, travaillant à la pression atmosphérique, à une pression supérieure ou une pression inférieure.
